(19) Europäisches Patentamt
European Patent Office
Office européen des brevets

(11) Numéro de publication: **0 299 867 B1**

(12) # FASCICULE DE BREVET EUROPEEN

(45) Date de publication du fascicule du brevet: **07.11.90**

(51) Int. Cl.[5]: **C07C 15/16**, C07C 2/86

(21) Numéro de dépôt: **88401814.4**

(22) Date de dépôt: **12.07.88**

(54) **Compositions d'oligomères de polyarylalcanes contenant des motifs xylène et leur procédé de fabrication.**

(30) Priorité: **16.07.87 FR 8710068**

(43) Date de publication de la demande: **18.01.89 Bulletin 89/3**

(45) Mention de la délivrance du brevet: **07.11.90 Bulletin 90/45**

(84) Etats contractants désignés: **AT BE CH DE ES FR GB GR IT LI LU NL SE**

(56) Documents cités: **EP-A- 0 136 230**

(73) Titulaire: **ATOCHEM, 4 & 8, Cours Michelet La Défense 10, F-92800 Puteaux(FR)**

(72) Inventeur: **Commandeur, Raymond, Le Rocher Avenue de Venaria, F-38220 Vizille(FR)**
Inventeur: **Gurtner, Bernard, 5 Boulevard Agutte Sambat, F-38000 Grenoble(FR)**

EP 0 299 867 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles compositions d'oligomères de polyarylalcanes contenant des motifs xylene et leur procédé de fabrication.

Ces produits sont utiles en tant que solvant de microencapsulation pour papier transfert sans carbone.

L'homme de l'art a déjà proposé dans le brevet EP 136.230 des mélanges d'oligomères sur base de benzyltoluène, dibenzyltoluène et de ditolylphenylméthane qui présentent l'avantage, par rapport à ces mêmes oligomères pris séparément, de cristalliser à très basse température et d'avoir une viscosité toujours compatible avec leur application comme diélectrique pour condensateurs. Ces caractéristiques les rend tout particulièrement aptes à une utilisation en tant que solvant de microencapsulation pour papier transfert sans carbone ; ils possèdent néammoins une caractéristique rédhibitoire pour un tel emploi, à savoir une odeur fortement désagréable qui se révèle tout comme la couleur au moment de la frappe sur machine à écrire, par exemple. Les compositions selon l'invention présentent non seulement une viscosité compatible avec l'application, mais se caractérisent de plus par une "absence d'odeur" absolument indispensable dans l'application visée.

Le brevet GB 1.346.364 résume les propriétés que doivent concilier les solvants de microencapsulation :

- solubiliser le colorant
- ne pas se vaporiser à la mise en oeuvre des microcapsules
- être inerte vis à vis du matériau d'encapsulation
- ne pas réagir avec le révélateur du colorant
- avoir une viscosité faible et peu sensible à la température
- ne pas avoir d'odeur désagréable.

Les inconvénients des mono et dibenzylalkylbenzène étant aussi connus de longue date, de nombreux mélanges de remplacement ont été proposés à partir d'oligomères de polyarylalcanes. Il est cependant toujours difficile de concilier la qualité des propriétés des produits obtenus avec l'aspect économique des moyens d'obtention de ce produit de remplacement.

C'est ainsi que dans le brevet US 3 939 095 est décrit le méthyl phényl -(2,5 dimethylphényl)méthane ou xylyl-2 paraxylène, dont la synthèse peut être réalisée par réaction du chlorure de méthylbenzyle sur le paraxylène suivie d'une séparation pour accéder à cet oligomère pur.

Enfin, dans la demande de brevet japonais JP-KOKAI 73-86 612 est cité l'utilisation en tant que solvant de microencapsulation du di(propylbenzyl)propylbenzène dont l'obtention fait appel à plusieurs étapes avec des purifications à chaque stade, et pose donc en plus de l'aspect économique, et tout comme le brevet précédent, des problèmes de rejet d'effluents et d'environnement.

De façon générale, on n'a pas réussi à mettre au point des produits particulièrement adaptés pour l'application solvant de microencapsulation, par exemple, du type monoxylylxylène qu'en utilisant des synthèses peu sélectives pour l'obtention de ces produits.

La présente invention propose des produits adaptés pour l'application solvant de microencapsulation et qui présentent l'avantage de pouvoir être fabriqués de façon simple.

La présente invention concerne des compositions d'oligomères de polyarylalcanes constituées du mélange d'au moins un oligomère A et au moins un oligomère B caractérisés en ce que :

- l'oligomère A est un mélange d'isomères de formule :

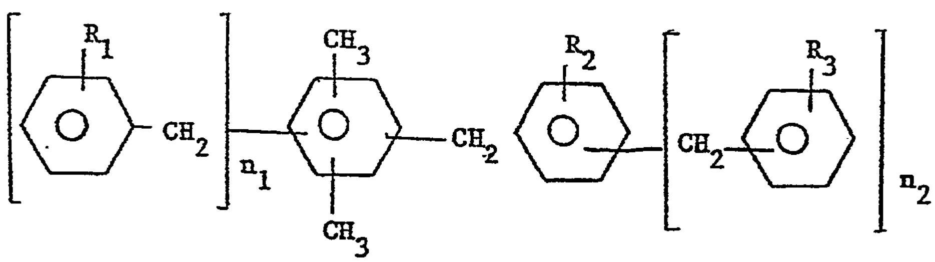

dans laquelle :

. $R_1$, $R_2$ et $R_3$ sont identiques ou différents et représentent H ou $CH_3$
. $n_1$ et $n_2$ = 0,1 et 2 avec $n_1 + n_2 \leq 3$
. chacun des isomères A pouvant avoir des substituants $R_1$, $R_2$, $R_3$ différents
- l'oligomère B est un mélange d'isomères de formule :

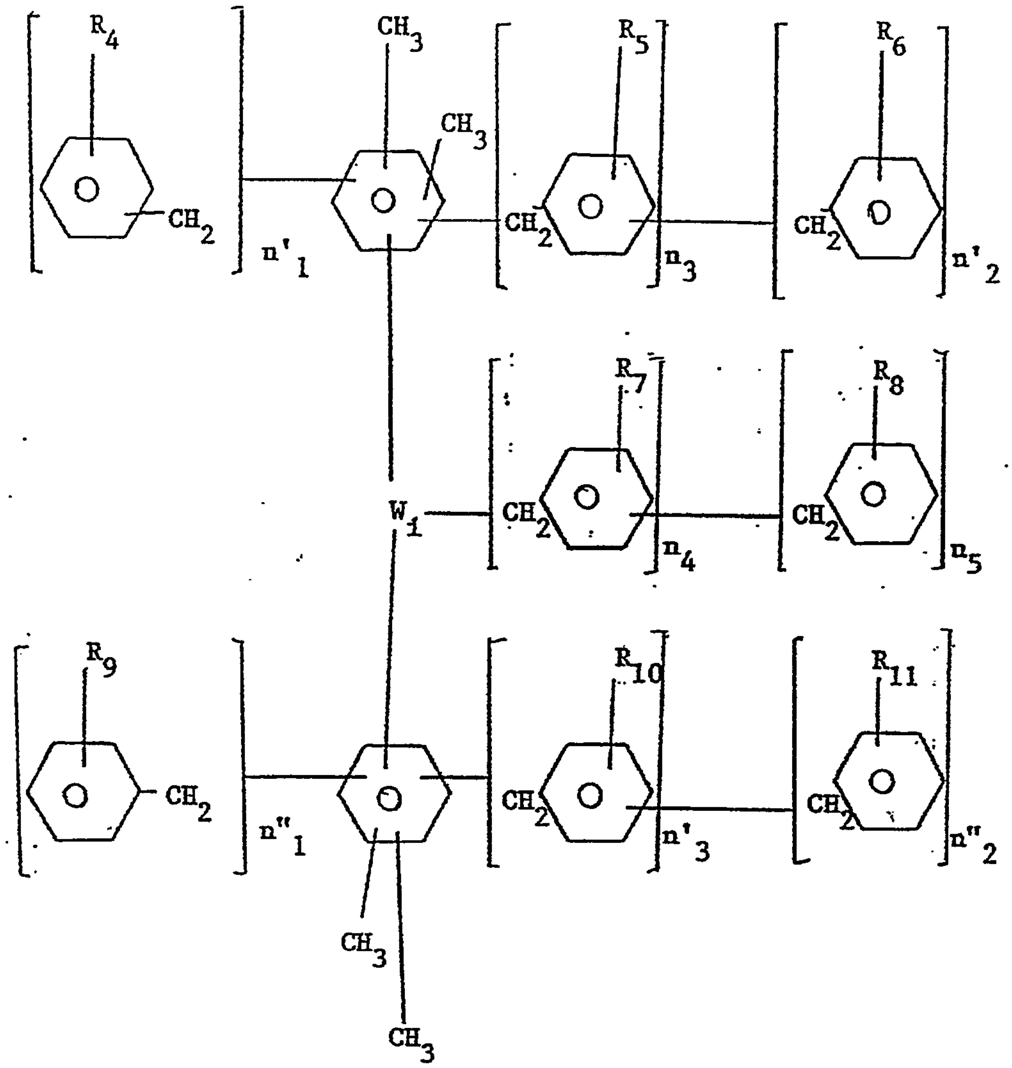

dans laquelle :

. $R_4$, $R_5$, $R_6$, $R_7$, $R_8$, $R_9$, $R_{10}$, $R_{11}$ sont identiques ou différents et représentent H ou $CH_3$,

. $n'_1$, $n''_1$ et $n_4$ = 0, 1 ou 2,

. $n'_2$, $n''_2$, $n_3$, $n'_3$ et $n_5$ = 0 ou 1,

. $n'_1 + n''_1 + n'_2 + n''_2 + n_3 + n'_3 + n_4 + n_5 \leqq 2$,

. i vaut 1 ou 2,

. $W_i$ est un groupe de liaison trivalent tel que :

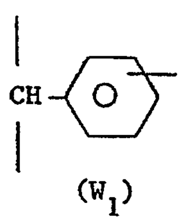

$(W_1)$

et/ou

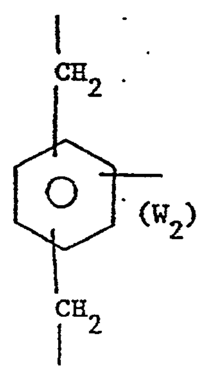

$(W_2)$

et dont les liaisons vers les groupes

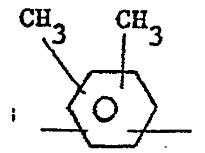

sont assurées par des atomes de carbone n'appartenant pas au groupement phényle de $W_i$.
. chacun des isomères B pouvant avoir des substituants $R_4$ à $R_{11}$ différents.

C'est-à-dire que B peut être un mélange des produits de formule (I) et (II), chacun des produits (I) et (II) étant lui-même un mélange d'isomères.

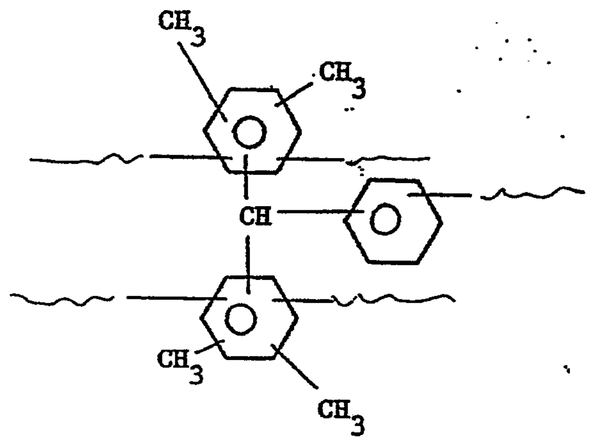

(I)

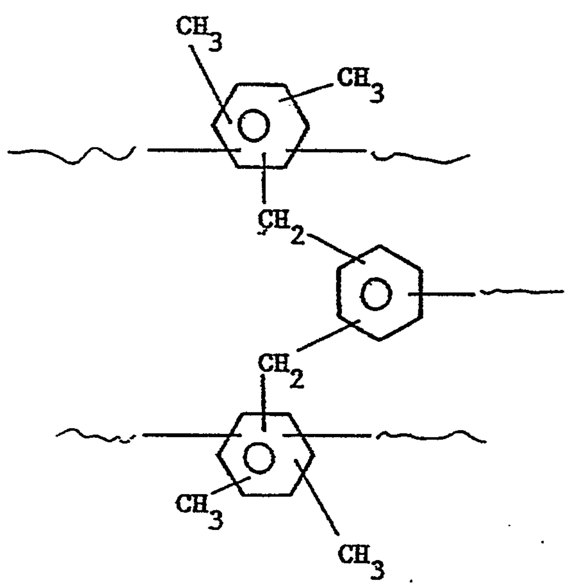

(II)

On utilise avantageusement les compositions dans lesquelles $R_1$ à $R_{11}$ sont des groupements $CH_3$.
On utilise aussi avantageusement les compositions dans lesquelles $W_i$ est

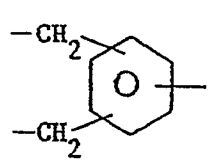

Les compositions préférées sont telles que :
- $R_1$ à $R_{11}$ sont des $CH_3$ et $W_i$ est

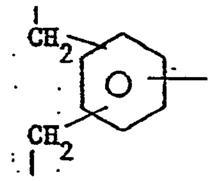

Les compositions selon l'invention présentent toutes les propriétées que doivent concilier les solvants de microencapsulation et la demanderesse a constaté avec surprise que ces produits ne sont pas odorants.

Les compositions selon l'invention peuvent être obtenues en mettant en oeuvre un procédé ne faisant appel en tant que matières premières qu'au chlore, au toluène et au xylène.

Un procédé de fabrication des compositions d'oligomères de polyarylalcanes selon l'invention est caractérisé en ce que, dans une première étape on fait réagir le chlore sur du toluène ou du xylène ou un mélange de toluène et de xylène en présence de générateur de radicaux, puis en élimine le toluène éventuel non réagi, et en ce que dans une seconde étape on soumet le produit de réaction de cette première étape à l'action d'un halogénure minéral ou d'un acide minéral en présence de xylène.

Dans la première étape l'hydrocarbure de départ est soit du xylène, soit un mélange de toluène et de xylène.

La chloration radicalaire de l'hydrocarbure est habituellement réalisée à une température comprise entre 50 et 110°C et mieux entre 70 et 100°C. Elle est de préférence menée de telle sorte qu'on ne transforme que 10 à 50 %, exprimé en pourcentage molaire, de l'hydrocarbure engagé en dérivé chloré correspondant. Le toluène non réagi est ensuite éliminé par exemple par distillation. En tant que générateur de radicaux libres, on peut employer soit une initiation photochimique, soit un initiateur chimique ; parmi les initiateurs chimiques, on peut citer les composés azo comme l'azodiisobutyronitrile ou encore l'azodivalé-ronitrile, les péroxydes comme par exemple le péroxyde de lauroyle. La quantité d'initiateur chimique mise en oeuvre est généralement comprise entre 0,05 et 3 % en poids par rapport à l'hydrocarbure engagé, et de préférence entre 0,1 et 1,5 %.

Le milieu réactionnel obtenu durant la première étape est ensuite en présence de xylène soumis à l'action d'un halogénure minéral, ou encore d'un acide minéral. Cette réaction a lieu en pratique à une température comprise entre 30 et 140°C, et de préférence entre 50 et 120°C.

Si l'hydrocarbure lors de la première étape contient du xylène, il n'est pas nécessaire d'en ajouter lors de la seconde étape.

Avantageusement on ajoute du xylène dans le milieu obtenu en fin de première étape après l'élimination du toluène éventuel.

De préférence on ajoute autant de moles de xylène que de toluène éventuel éliminé.

Parmi les halogénures minéraux, on peut utiliser le chlorure ferrique, le trichlorure d'antimoine, le tétrachlorure de titane ou encore le chlorure d'aluminium à des teneurs pondérales par rapport au milieu réactionnel compris habituellement entre 50 ppm et 1 % et de préférence entre 100 ppm et 0,5 %. Les acides minéraux peuvent également être utilisés : l'acide sulfurique par exemple à une concentration pondérale comprise entre 70 et 95 %. Il est aussi possible d'employer les zeolithes ou encore certains oxydes minéraux. Une variante du procédé au niveau de cette deuxième étape consiste à couler le mélange réactionnel de la première étape dans le xylène, ou xylène et le mélange d'oligomères selon l'invention, contenant l'halogénure ou l'acide minéral sous forme de solution ou de dispersion ; cette variante est particulièrement intéressante pour la mise en continu d'un tel procédé car il est bien entendu que cette synthèse est réalisable en discontinu et en continu.

On peut, après distillation du xylène en excès, procéder à l'élimination de l'halogénure minéral ou de l'acide minéral par toute technique connue telle que : lavage à l'eau, neutralisation, séchage.

On utilise avantageusement dans la première étape le xylène, les groupes $R_1$ à $R_{11}$ (dans les compositions selon l'invention) sont alors des méthyles, et $W_i$ est

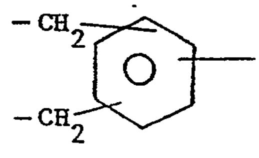

Le xylène utilisé peut être le mélange des isomères ou les isomères pris séparément, ou encore des combinaisons 2 à 2.

Selon le procédé décrit on obtient, de façon courante, directement le mélange d'oligomères de polyarylalcanes dans les proportions pondérales suivantes :

Composé A en mélange d'isomères : $n_1 + n_2 = 0$ compris entre 56 et 90 %
$n_1 + n_2 = 1$ compris entre 7 et 28 %
$n_1 + n_2 = 2$ compris entre 1,5 et 8 %
$n_1 + n_2 = 3$ compris entre 0,1 et 2 %

Composé B en mélange d'isomères : $n'_1 + n''_1 + n'_2 + n''_2 + n_3 + n'_3 + n_4 + n_5 = 0$ compris entre 1,1 et 10 %
$n'_1 + n''_1 + n'_2 + n''_2 + n_3 + n'_3 + n_4 + n_5 = 1$ compris entre 0,25 et 3 %
$n'_1 + n''_1 + n'_2 + n''_2 + n_3 + n'_3 + n_4 + n_5 = 2$ compris entre 0,05 et 1 %

Suivant l'usage applicatif du mélange d'oligomères de polyarylalcanes selon l'invention, il peut être intéressant de procéder à une évaporation-flash de ce mélange pour éliminer les traces d'impuretés provenant soit des matières premières, soit du procédé, ou ayant une origine accidentelle ; dans tous les cas, leurs teneurs pondérales ne dépassent pas 1 à 2 %. Parmi les appareils utilisables, on préfèrera un évaporateur à film mince ; il faut signaler cependant qu'au niveau industriel, les possibilités techniques de tels appareils au niveau de la tenue au vide ne permettent pas toujours de récupérer l'intégralité du mélange d'oligomères de polyarylalcanes : ces produits évaporés font néanmoins partie intégrante de l'invention, comme c'est le cas en particulier des isomères du composé A pour $n_1 + n_2 = 3$ et du composé B pour $n'_1 + n''_1 + n'_2 + n''_2 + n_3 + n'_3 + n_4 + n_5 = 2$.

Les mélanges d'oligomères sont obtenus, selon le procédé décrit, avec des rendements pondéraux exprimés par rapport aux hydrocarbures réagis pouvant même atteindre 98 % dans les meilleures conditions.

Les exemples suivants illustrent l'invention sans la limiter.

EXEMPLE 1

Dans un réacteur muni d'une agitation d'un condenseur, d'un tube d'alimentation de chlore et d'une lampe PHILIPS TLADK de 30 Watt, on place 424 g d'orthoxylène (4 moles) ; on introduit ensuite 71 g de chlore gazeux (1 mole) en maintenant la température à 80°C durant 1 heure.

Après arrêt de l'initiation photochimique, le milieu réactionnel est placé dans une ampoule de coulage et il est introduit en 1 heure dans un réacteur muni d'une agitation contenant 2 moles d'orthoxylène et 60 mg de $FeCl_3$ à une température de 100°C. L'ensemble est maintenu encore 1 heure à 100°C sous agitation après fin de coulage. Le milieu réactionnel, après refroidissement, est lavé à l'acide chlorhydrique 10 %, puis à l'eau jusqu'à neutralité. L'excès d'orthoxylène est éliminé par distillation sous vide de 10 mm de mercure (1330 Pa) avec une colonne de quelques plateaux de manière à ce que la teneur résiduelle en o-xylène dans le produit en pied soit en inférieure à 500 ppm (température de pied en fin de distillation = 190°C).

Le mélange d'oligomères de polyarylalcanes obtenu est tel que dans la formule générale $R_1$ à $R_{11}$ sont des $CH_3$ et $W_i$ est :

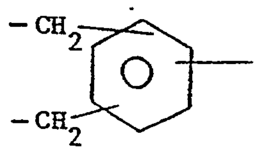

Le mélange d'oligomères de polyarylalcanes obtenu a la composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 73 | 14,6 | 3,7 | 1,7 | – | – | – |
| B | – | – | – | – | 5,4 | 1,3 | 0,3 |

Le rendement pondérale calculé d'après l'o-xylène consommé est de 97 %.

Ce produit soumis à une évaporation flash à 300°C sous 0,5 mm de Hg (66 Pa), donne avec un rendement de 96 % un mélange d'oligomères de polyarylalcanes très faiblement odorant, dont la seule différence avec la composition donnée ci-dessus est l'absence des produits correspondants à :

A et $n_1 + n_2 = 3$

B et $n'_1 + n''_1 + n'_2 + n''_2 + n_3 + n'_3 + n_4 + n_5 = 2$

La viscosité à:

20°C est 34,4 cps (34,4 m.Pa.s)

40°C est 12,3 cps (12,3 m.Pa.s)

EXEMPLE 2

En opérant dans les mêmes conditions de l'exemple 1, mais en utilisant 6 moles d'o-xylène (592 g) à la photochloration pour 1 mole de chlore introduit et 4 moles d'o-xylène à la réaction de couplage. Le mélange d'oligomères de polyarylalcanes obtenu a la même formule que dans l'exemple 1 et a la composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 82 | 8,3 | 3,4 | 1,3 | – | – | – |
| B | – | – | – | – | 4 | 0,8 | 0,2 |

Le rendement pondéral calculé d'après l'o-xylène réagi est de 97 %.

La viscosité est la suivante :

40°C = 9,9 cps

20°C = 25,8 cps

Ce produit soumis à une évaporation flash à 300°C sous 0,5 mm de Hg (66 Pa) donne avec un rendement de 98 % un mélange d'oligomères de polyarylalcanes très faiblement odorant dont la composition diffère avec celle donnée ci-dessus par l'absence des produits correspondants à :

A et $n_1 + n_2 = 3$

B et $n'_1 + n''_1 + n'_2 + n''_2 + n_3 + n'_3 + n_4 + n_5 = 2$

Pour une viscosité à :

20°C = 18,4 cps (18,4 m.Pa.s)

40°C = 7,6 cps (7,6 m.Pa.s)

EXEMPLE 3

En opérant dans les mêmes conditions que l'exemple 1 mais en utilisant 2 moles d'orthoxylène à la photochloration pour 1 mole de chlore introduit et en conservant 2 moles d'oxylène à la réaction de couplage.

Le mélange d'oligomères de polyarylalcanes obtenu a la même formule que dans l'exemple 1 et a composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 63 | 17,3 | 7,2 | 1,3 | - | - | - |
| B | - | - | - | - | 8,2 | 2,6 | 1 |

Le rendement pondéral d'après l'orthoxylène réagi est de 98 %.

Ce produit soumis à une évaporation flash à 300°C sous 0,5 mm de Hg (66 Pa) donne avec un rendement de 98 %, un mélange d'oligomères de polyarylalcanes très faiblement odorant dont la composition diffère avec celle donnée ci-dessus par l'absence des produits correspondants à :

A et $n_1 + n_2 = 3$

B et $n'_1 + n''_1 + n'_2 + n''_2 + n_3 + n'_3 + n_4 + n_5 = 2$

Pour une viscosité à :

20°C = 48,1 cps (48,1 m.Pa.s)

40°C = 19,6 cps (19,6 m.Pa.s)

EXEMPLE 4

Identique à l'exemple 1 mais en remplaçant l'orthoxylène par le paraxylène.

Le mélange d'oligomères de polyarylalcanes obtenu faiblement odorant, a la même formule que dans l'exemple 1 et a la composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 65 | 20,5 | 4,5 | 1,9 | - | - | - |
| B | - | - | - | - | 6 | 1,8 | 0,3 |

Le rendement pondéral d'après le paraxylène réagi est de 98 %.

EXEMPLE 5

Identique à l'exemple 1 mais en remplaçant l'orthoxylène par le métaxylène.

Le mélange d'oligomères de polyarylalcanes obtenu faiblement odorant a la même formule que dans l'exemple 1 et a la composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 68 | 18,5 | 4,2 | 1,8 | - | - | - |
| B | - | - | - | - | 5,7 | 1,5 | 0,3 |

Le rendement pondéral d'après le métaxylène réagi est de 98 %.

## Revendications

1. Compositions d'oligomères de polyarylalcanes constituées du mélange d'au moins un oligomère A et d'au moins un oligomère B caractérisés en ce que :
- l'oligomère A est un mélange d'isomères de formule :

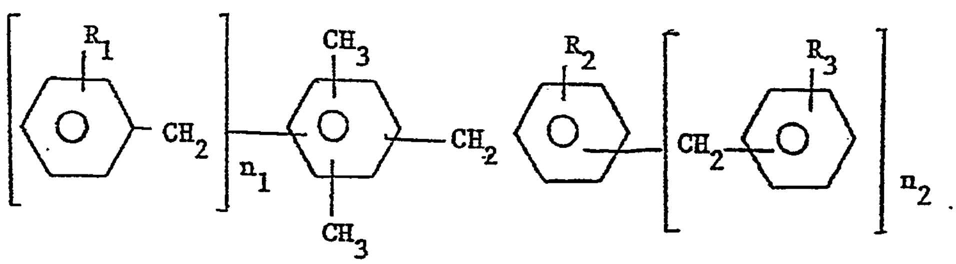

dans laquelle :
. $R_1$, $R_2$ et $R_3$ sont identiques ou différents et représentent H ou $CH_3$
. $n_1$ et $n_2$ = 0, 1 et 2 avec $n_1 + n_2 \leq 3$
. chacun des isomères A pouvant avoir des substituants $R_1$, $R_2$ et $R_3$ différents :
- l'oligomère B est un mélange d'isomères de formule :

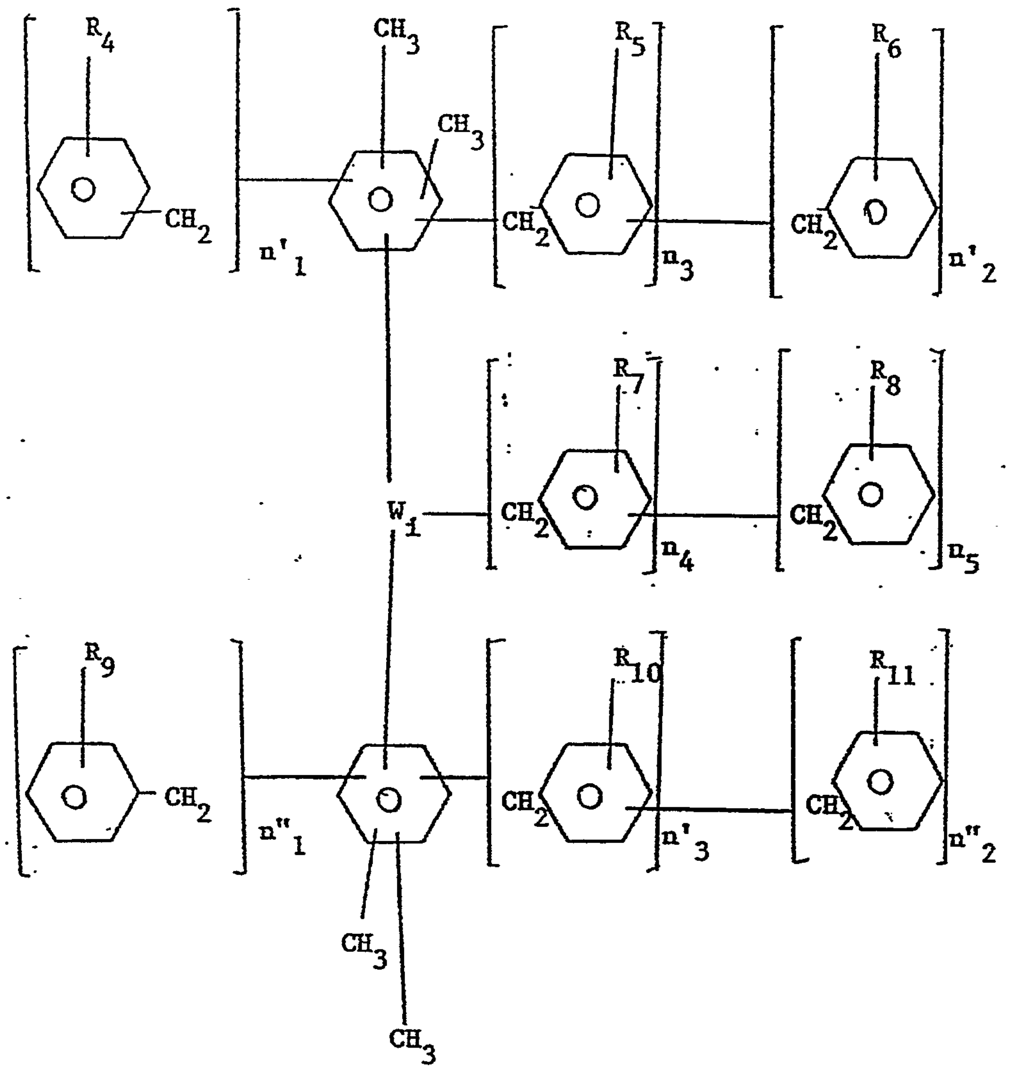

dans laquelle :

. $R_4$, $R_5$, $R_6$, $R_7$, $R_8$, $R_9$, $R_{10}$, $R_{11}$ sont identiques ou différents et représentent H ou $CH_3$,
. $n'_1$, $n''_1$ et $n_4$ = 0,1 ou 2,
. $n'_2$, $n''_2$, $n_3$, $n'_3$ et $n_5$ = 0 ou 1,
. $n'_1 + n''_1 + n'_2 + n''_2 + n_3 + n'_3 + n_4 + n_5 \leqq 2$,
. i vaut 1 ou 2,
. $W_i$ est un groupe de liaison trivalent tel que :

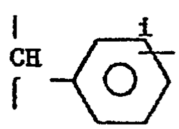

(W1) et/ou

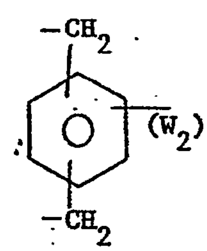

(W2)

et dont les liaisons vers les groupes

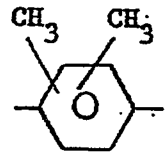

sont assurées par des atomes de carbone n'appartenant pas au groupement phényle de $W_i$.
. chacun des isomères B pouvant avoir des substituants $R_4$ à $R_{11}$ différents

2. Compositions selon la revendication 1 caractérisées en ce que $R_1$ à $R_{11}$ sont des groupements $CH_3$.

3. Compositions selon les revendications 1 ou 2 caractérisées en ce que $W_i$ est:

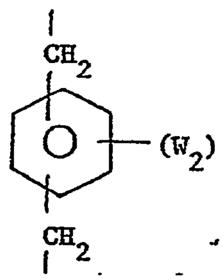

4. Compositions selon l'une des revendications 1 à 3 caractérisées en ce que le mélange d'oligomères se trouve dans les proportions pondérales de :

Composé A en mélange d'isomères : $n_1 + n_2 = 0$ compris entre 56 et 90 %
$n_1 + n_2 = 1$ compris entre 7 et 28 %
$n_1 + n_2 = 2$ compris entre 1,5 et 8 %
$n_1 + n_2 = 3$ compris entre 0,1 et 2 %

Composé B en mélange d'isomères : $n'_1 + n''_1 + n'_2 + n''_2 + n_3 + n'_3 + n_4 + n_5 = 0$ compris entre 1,1 et 10 %
$n'_1 + n''_1 + n'_2 + n''_2 + n_3 + n'_3 + n_4 + n_5 = 1$ compris entre 0,25 et 3 %

5. Procédé de fabrication des compositions d'oligomères de polyarylalcanes selon la revendication 1 caractérisé en ce que dans une première étape on fait réagir le chlore sur le toluène ou sur le xylène ou un mélange de toluène et de xylène en présence de générateur de radicaux, puis on élimine le toluène éventuel non réagi et en ce que dans une seconde étape on soumet le produit de réaction de cette première étape à l'action d'un halogénure minéral ou d'un acide minéral en présence de xylène.

6. Procédé selon la revendication 5 caractérisé en ce que dans la première étape on ne transforme en dérivé chloré que 10 à 50 % molaire du toluène ou du xylène ou du mélange de toluène et de xylène engagé.

7. Procédé selon les revendications 5 ou 6, caractérisé en ce que dans la première étape on fait régir le chlore sur le xylène en présence de générateur de radicaux et en ce que dans une seconde étape on soumet le produit de réaction de cette première étape contenant du xylène non réagi à l'action d'un halogénure minéral ou d'un acide minéral.

8. Procédé selon l'une des revendications 5 à 7, caractérisé en ce qu'on procède à une évaporation flash sur le produit obtenu en seconde étape.

## Patentansprüche

1. Massen auf der Basis von Polyarylalkan-Oligomeren bestehend aus dem Gemisch aus mindestens einem Oligomeren A und mindestens einem Oligomeren B, dadurch gekennzeichnet, daß das
– Oligomere A ein Gemisch von Isomeren der Formel

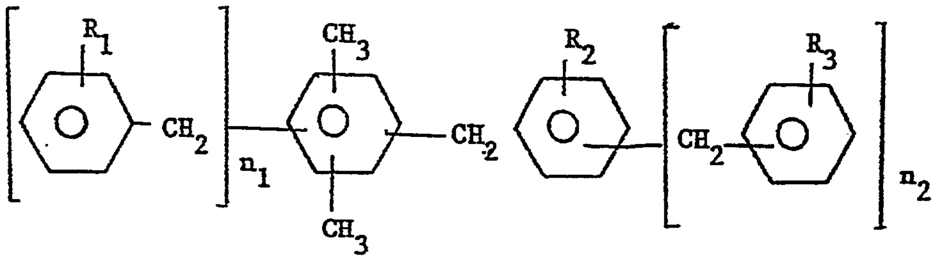

ist, in der
$R_1$, $R_2$ und $R_3$ gleich oder verschieden sind und für H oder für $CH_3$ stehen, $n_1$ und $n_2$ = 0,1 oder 2 sind, wobei $n_1 + n_2 \leq 3$ gilt, jedes der Isomeren A unterschiedliche Substituenten $R_1$, $R_2$ und $R_3$ haben kann,
– das Oligomere B ein Gemisch von Isomeren der Formel

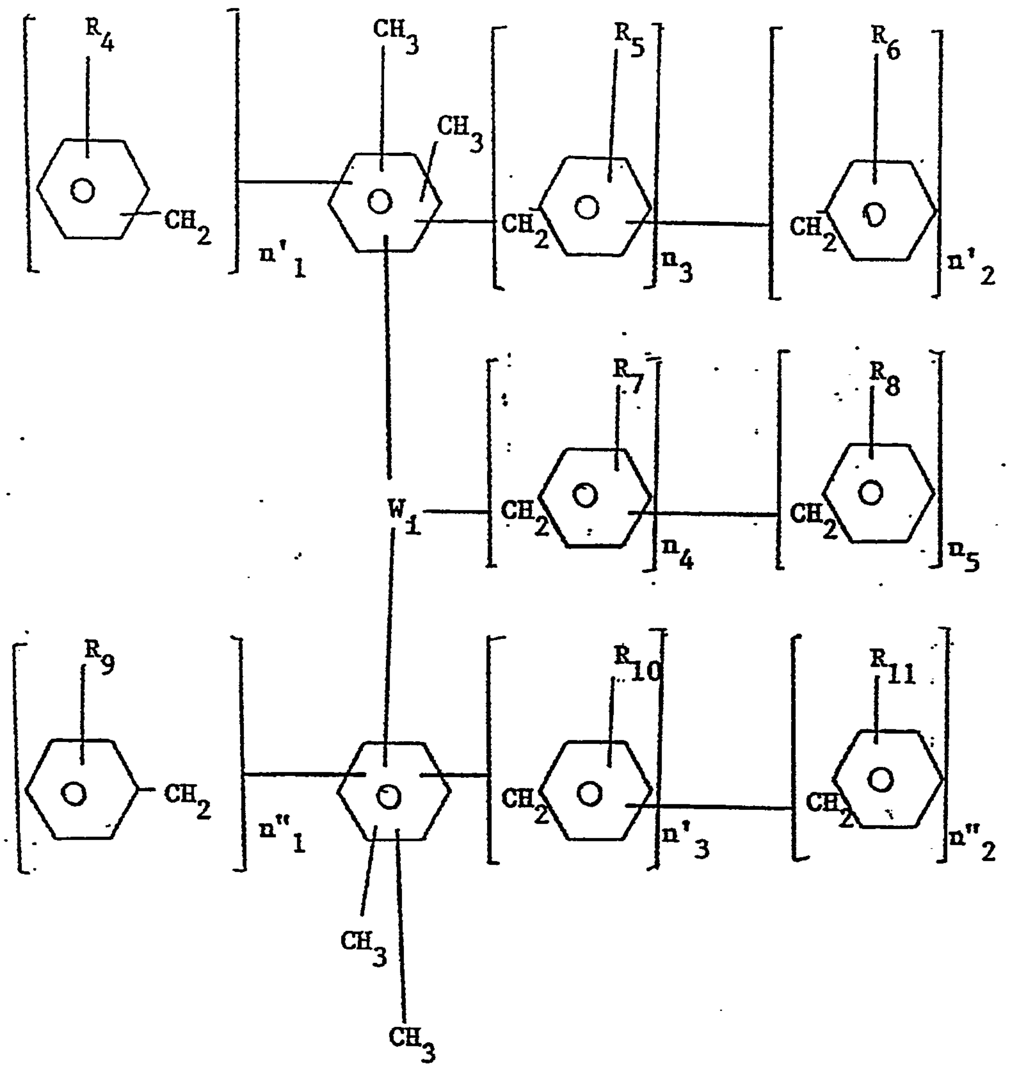

ist, in der $R_4$, $R_5$, $R_6$, $R_7$, $R_8$, $R_9$, $R_{10}$ und $R_{11}$ gleich oder verschieden sind und H oder $CH_3$ bedeuten,
$n'_1$, $n''_1$ und $n_4$ = 0,1 oder 2 sind,
$n'_2$, $n''_2$, $n_3$, $n'_3$ und $n_5$ = 0 oder 1 sind,
$n'_1 + n''_1 + n'_2 + n''_2 + n_3 + n'_3 + n_4 + n_5 \leqq 2$ ist
i die Bedeutung 1 oder 2 hat, $W_i$ eine dreiwertige Bindungsgruppe ist, derart, daß:,

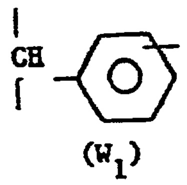

und/oder

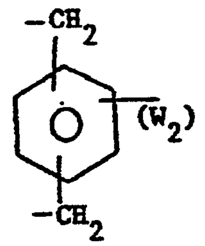

und deren Bindungen zu den Gruppen

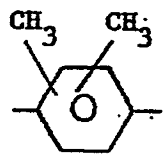

durch Kohlenstoffatome sichergestellt sind, die nicht der Phenylgruppe von $W_i$ angehören, jedes der Isomeren B
unterschiedliche Substituenten $R_4$ bis $R_{11}$ haben kann.

2. Massen nach Anspruch 1, dadurch gekennzeichnet, daß $R_i$ bis $R_{11}$ $CH_3$-Gruppen sind.

3. Massen nach Anspruch 1 oder 2, dadurch gekennzeichnet, daß $W_i$ die Bedeutung

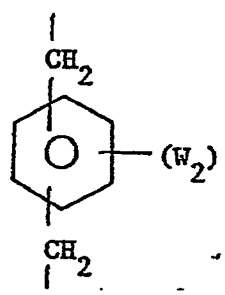

hat.

4. Massen nach einem der Ansprüche 1 bis 3, dadurch gekennzeichnet, daß das Oligomerengemisch folgenden Gewichtsanteilen entspricht:

Verbindung A als Isomerengemisch:
$n_1 + n_2 = 0$ im Bereich von 56 bis 90%
$n_1 + n_2 = 1$ im Bereich von 7 bis 28%
$n_1 + n_2 = 2$ im Bereich von 1,5 bis 8%
$n_1 + n_2 = 3$ im Bereich von 0,1 bis 2%

Verbindung B als Isomerengemisch:
$n'_1 + n''_1 + n'_2 + n''_2 + n_3 + n'_3 + n_4 + n_5 = 0$ im Bereich von 1,1 bis 10 %
$n'_1 + n''_1 + n'_2 + n''_2 + n_3 + n'_3 + n_4 + n_5 = 1$ im Bereich von 0,25 bis 3 %
$n'_1 + n''_1 + n'_2 + n''_2 + n_3 + n'_3 + n_4 + n_5 = 2$ im Bereich von 0.05 bis1%

5. Verfahren zur Herstellung von Massen auf der Basis von Polyarylalkan-Oligomeren nach Anspruch 1, dadurch gekennzeichnet, daß man in einer ersten Stufe Chlor auf Toluol oder auf Xylol oder auf ein Gemisch aus Toluol und Xylol in Gegenwart eines Radikalbildners einwirken läßt, darauf das gegebenenfalls nicht umgesetzte Toluol abtrennt und daß man in einer zweiten Stufe das Reaktionsprodukt

aus der ersten Stufe der Einwirkung eines anorganischen Halogenids oder einer anorganischen Säure in Gegenwart von Xylol unterwirft.

6. Verfahren nach Anspruch 5, dadurch gekennzeichnet, daß man in der ersten Stufe nur 10 bis 50 Mol-% des eingesetzten Toluols oder Xylols oder Gemisches aus Toluol und Xylol in ein Chlorderivat überführt.

7. Verfahren nach den Ansprüchen 5 oder 6, dadurch gekennzeichnet, daß man in der ersten Stufe Chlor auf Xylol in Gegenwart eines Radikalbildners einwirken läßt und daß man in einer zweiten Stufe das Reaktionsprodukt aus der ersten Stufe, das nicht umgesetztes Xylol enthält, der Einwirkung eines anorganischen Halogenids oder einer anorganischen Säure unterwirft.

8. Verfahren nach einem der Ansprüche 5 bis 7, dadurch gekennzeichnet, daß man mit dem in der zweiten Stufe erhaltenen Produkt eine Schnellverdampfung durchführt.

**Claims**

1. Compositions of oligomers of polyarylalkanes consisting of the mixture of at least one oligomer A and at least one oligomer B, characterized in that:
– the oligomer A is a mixture of isomers of formula:

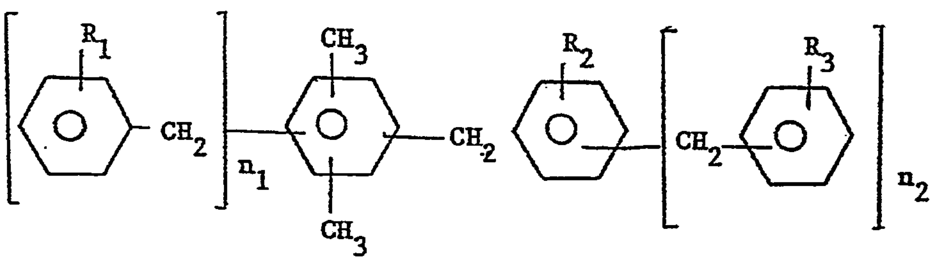

in which:
$R_1$, $R_2$ and $R_3$ are identical or different and represent H or $CH_3$, and
$n_1$ and $n_2$ = 0, 1 and 2 with $n_1 + n_2 \leq 3$
each of the isomers A being able to have different substituents $R_1$, $R_2$ and $R_3$:
– the oligomer B is a mixture of isomers of formula:

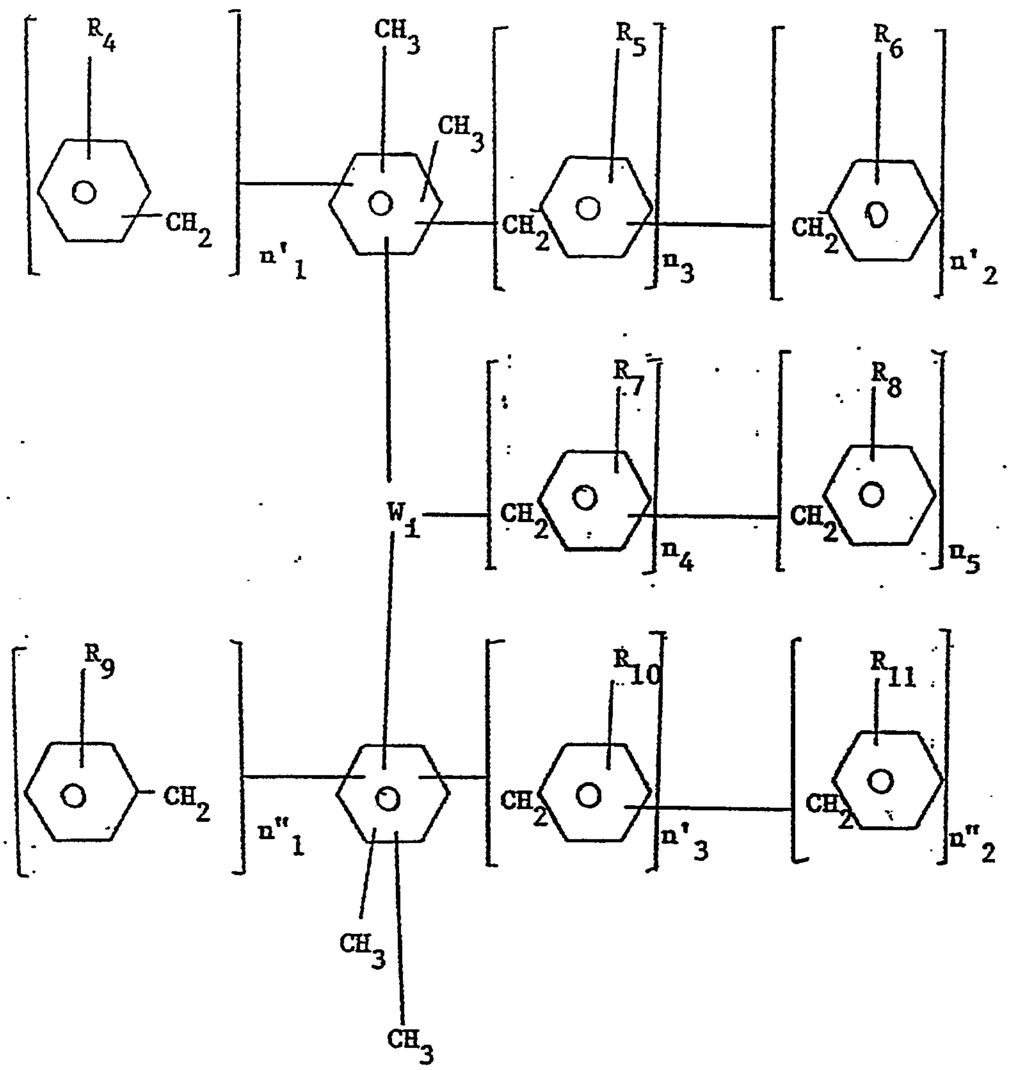

in which:
$R_4$, $R_5$, $R_6$, $R_7$, $R_8$, $R_9$, $R_{10}$ and $R_{11}$ are identical or different and represent H or $CH_3$,
$n'_1$, $n''_1$ and $n_4$ = 0,1 or 2,
$n'_2$, $n''_2$, $n_3$, $n'_3$ and $n_5$ = 0 or 1,
$n'_1 + n''_1 + n'_2 + n''_2 + n_3 + n'_3 + n_4 + n_5 \leq 2$
i is 1 or 2, and $W_1$ is a group of containing a trivalint bond, such as:

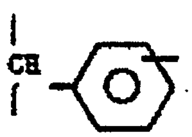

($W_1$)

and/or

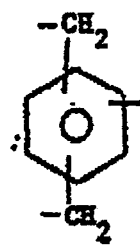

($W_2$)

and in which the bonds towards the groups

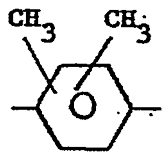

are ensured by carbon atoms not belonging to the phenyl group of $W_i$, each of the isomers B being able to have different substituents $R_4$ to $R_{11}$.

2. Compositions according to Claim 1, characterized in that $R_1$ to $R_{11}$ are $CH_3$ groups.

3. Compositions according to Claims 1 or 2, characterized in that $W_i$ is:

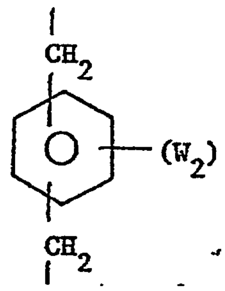

4. Compositions according to one of Claims 1 to 3, characterized in that the mixture of oligomers is in the proportions by weight of:

Compound A as a mixture of isomers:
$n_1 + n_2 = 0$ of between 56 and 90%
$n_1 + n_2 = 1$ of between 7 and 28%
$n_1 + n_2 = 2$ of between 1.5 and 8%
$n_1 + n_2 = 3$ of between 0.1 and 2%, and

Compound B as a mixture of isomers:
$n'_1 + n''_1 + n'_2 + n''_2 + n_3 + n'_3 + n_4 + n_5 = 0$ of between 1,1 and 10 %
$n'_1 + n''_1 + n'_2 + n''_2 + n_3 + n'_3 + n_4 + n_5 = 1$ of between 0,25 and 3 %
$n'_1 + n''_1 + n'_2 + n''_2 + n_3 + n'_3 + n_4 + n_5 = 2$ of between 0.05 and 1%

5. Process for the production of compositions of oligomers of polyarylalkanes according to Claim 1, characterized in that in a first step chlorine is reacted with toluene or with xylene or mixture of toluene and xylene in the presence of a compound generating free radicals and any unreacted toluene is then removed and in that in a second step the reaction product from this first step is subjected to the action of an inorganic halide or of a mineral acid in the presence of xylene.

6. Process according to Claim 5, characterized in that only 10 to 50 molar % of the toluene or of the xylene or of the mixture of toluene and xylene employed is converted to the chloro derivative in the first step.

7. Process according to Claim 5 ot 6, characterized in that in the first step chlorine is reacted with xylene in the presence of a compound generating free radicals and in that in a second step the reaction product from this first step, containing unreactive xylene, is subjected to the action of an inorganic halide or of a mineral acid.

8. Process according to one of Claims 5 to 7, characterized in that a flash evaporation is carried out on the product obtained in the second step.